(19) Europäisches Patentamt
European Patent Office
Office européen des brevets

(11) **EP 3 629 907 B1**

(12) # EUROPÄISCHE PATENTSCHRIFT

(45) Veröffentlichungstag und Bekanntmachung des Hinweises auf die Patenterteilung:
**13.03.2024   Patentblatt 2024/11**

(21) Anmeldenummer: **18728550.7**

(22) Anmeldetag: **22.05.2018**

(51) Internationale Patentklassifikation (IPC):
**A61B 5/021** $^{(2006.01)}$     **A61B 5/00** $^{(2006.01)}$
**A61B 5/024** $^{(2006.01)}$     **A61B 5/318** $^{(2021.01)}$

(52) Gemeinsame Patentklassifikation (CPC):
**A61B 5/6887; A61B 5/02108; A61B 5/02125;**
**A61B 5/02405; A61B 5/318; A61B 5/4035;**
A61B 5/486

(86) Internationale Anmeldenummer:
**PCT/EP2018/063406**

(87) Internationale Veröffentlichungsnummer:
**WO 2018/215482 (29.11.2018 Gazette 2018/48)**

(54) **VERFAHREN UND VORRICHTUNG ZUR ERMITTLUNG WENIGSTENS EINES BLUTDRUCKWERTES EINES PROBANDEN**

METHOD AND APPARATUS FOR DETERMINING AT LEAST ONE BLOOD PRESSURE VALUE OF A SUBJECT

MÉTHODE ET APPAREIL POUR DÉTERMINER AU MOINS UNE VALEUR DE PRESSION ARTERIELLE D'UN SUJET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.05.2017   AT 504342017**

(43) Veröffentlichungstag der Anmeldung:
**08.04.2020   Patentblatt 2020/15**

(73) Patentinhaber:
• **Human Research Institut für Gesundheitstechnologie und Präventionsforschung GmbH**
  **8160 Weiz (AT)**
• **Joysys GmbH**
  **8160 Weiz (AT)**
• **ams AG**
  **8141 Premstätten (AT)**

(72) Erfinder:
• **MOSER, Maximilian**
  **9074 Keutschach (AT)**
• **HASSLER, Thomas**
  **8045 Graz (AT)**
• **STOCKMEIER, Thomas**
  **8141 Premst tten (AT)**

• **GRUBER, Bernhard**
  **8112 Gratwein (AT)**

(74) Vertreter: **Gibler & Poth Patentanwälte KG**
  **Dorotheergasse 7/14**
  **1010 Wien (AT)**

(56) Entgegenhaltungen:
CN-B- 103 598 876      US-A1- 2010 125 212
US-A1- 2012 179 382

• SUJAY DEB ET AL: "Cuff-Less Estimation of Blood Pressure Using Pulse Transit Time and Pre-ejection Period", CONVERGENCE INFORMATION TECHNOLOGY, 2007. INTERNATIONAL CONFERENCE ON, IEEE, PISCATAWAY, NJ, USA, 21. November 2007 (2007-11-21), Seiten 941-944, XP031225325, ISBN: 978-0-7695-3038-3

- J MUEHLSTEFF ET AL: "Cuffless Estimation of Systolic Blood Pressure for Short Effort Bicycle Tests: The Prominent Role of the Pre-Ejection Period", ENGINEERING IN MEDICINE AND BIOLOGY SOCIETY (EMBC), 2013 35TH ANNUAL INTERNATIONAL CONFERENCE OF THE IEEE, Bd. 1, 1. August 2006 (2006-08-01), Seiten 5088-5092, XP055493570, ISSN: 1557-170X, DOI: 10.1109/IEMBS.2006.260275
- DILPREET BUXI ET AL: "A survey on signals and systems in ambulatory blood pressure monitoring using pulse transit time", PHYSIOLOGICAL MEASUREMENT, INSTITUTE OF PHYSICS PUBLISHING, BRISTOL, GB, vol. 36, no. 3, 19 February 2015 (2015-02-19), XP020280790, ISSN: 0967-3334, DOI: 10.1088/0967-3334/36/3/R1 [retrieved on 2015-02-19]
- David Campo ET AL: "Measurement of Aortic Pulse Wave Velocity With a Connected Bathroom Scale", American Journal of Hypertension, vol. 30, no. 9, 17 May 2017 (2017-05-17), pages 876-883, XP055492849, United States ISSN: 0895-7061, DOI: 10.1093/ajh/hpx059
- HYUN JAE BAEK ET AL: "Enhancing the estimation of blood pressure using pulse arrival time and two confounding factors", PHYSIOLOGICAL MEASUREMENT, INSTITUTE OF PHYSICS PUBLISHING, BRISTOL, GB, vol. 31, no. 2, 1 February 2010 (2010-02-01), pages 145-157, XP020175811, ISSN: 0967-3334

**Beschreibung**

Die Erfindung betrifft ein Verfahren zur Ermittlung wenigstens eines Blutdruckwertes eines Probanden gemäß Patentanspruch 1.

Der Blutdruck, als einer der wesentlichsten Kreislaufparameter, wird klassisch am Oberarm mittels einer Manschette gemessen. Dies ist zwar eine bekanntermaßen genaue Messmethode, stellt allerdings gewisse Anforderungen an den Messenden und ist für Laien kaum sinnvoll durchführbar.

Die CN 103598876 B zeigt ein Verfahren und System zur Ermittlung des systolischen und des diastolischen Blutdrucks einer Person, wobei in einem ersten Schritt ein Pulswellensignal und ein Elektrokardiogramm ermittelt werden.

Die Publikation Sujay Deb et. Al: "Cuff-Less Estimation of Blood Pressure using Pulse Transit Time and Pre-ejection Period"zeigt eine Methode zur Blutdruckbestimmung unter Einbeziehung der PTT und der PEP, wobei die PAT als das gemessene Zeitintervall von der R-Welle des EKG-Signals zum Fuß des PPG Signals definiert ist.

Die Publikation J. Muehlsteff. et. Al: "Cuffless Estimation of Systolic Blood Pressure for Short Effort Bicycle Tests: The Prominent Role oft he Pre-Ejection Period" offenbart spezifische Beiträge der PEP und der PTT für die Blutdruckmessung basierend auf der Pulswellenmethode.

Die US 2010/0125212 A1 offenbart ein Verfahren zur Überprüfung der Genauigkeit einer Blutdruckmessung einer Blutdruckmessvorrichtung. Der Benutzer kann physische Parameter wie Größe und Geschlecht in eine Eingabeeinrichtung einer Vorrichtung eingeben, wobei die Vorrichtung automatisch eine passende Armlänge aus einer Datenbank anhand der Größe des Benutzers auswählt.

Die US 2012/0179382 A1 zeigt ein System zur Ermittlung der Herzleistung, insbesondere zur Ermittlung ventrikulärer Arrhythmie. Es wird ein ermittelter Parameter mit einem Schwellenwert innerhalb eines Wertebereichs verglichen um einen Vergleichsindikator für einen medizinischen Zustand zur Verfügung zu stellen.

De Publikation Hyun Jae Beak et al: "Enhancing the stimation of blood pressure using pulse arrival time and two confounding factors" offenbart die Bestimmung eines Blutdruckwerts unter Berücksichtigung der Pulse Transit Time und der Pre-ejection Period.

Darüber hinaus sind weitere Methoden bekannt den Blutdruck zu ermitteln, wobei die Laufzeit einer Pulswelle vom Herzen zu einem peripheren Blutgefäß gemessen wird, und daraus auf den Blutdruck geschlossen wird. Nachteilig an den bislang bekannten derartigen Verfahren bzw. Vorrichtungen ist die erwiesenermaßen geringe Genauigkeit der derart ermittelten Blutdruckwerte. Kontrollmessungen haben ergeben, dass die Streuung derartiger "Messungen" mit bekannten Geräten derart ungenau sind, dass diese aus medizinischer Sicht aussagelos bzw. unbrauchbar sind.

Aufgabe der Erfindung ist es daher ein Verfahren der eingangs genannten Art anzugeben, mit welchem die genannten Nachteile vermieden werden können, mit welchem einfach und mit wenigen Messwerten eine genaue Ermittlung wenigstens eines Blutdruckwertes, insbesondere eines zeitlichen Verlaufes der Blutdruckwerte, eines Probanden möglich ist.

Erfindungsgemäß wird dies durch die Merkmale des Patentanspruches 1 erreicht.

Dadurch ist einfach eine genaue Ermittlung wenigstens eines Blutdruckwertes eines Probanden möglich. Durch die zusätzliche Berücksichtigung des vegetativen Tonus konnte die Genauigkeit deutlich verbessert werden. Es hat sich gezeigt, dass der vegetative Tonus erheblichen Einfluss auf den Verlauf des Blutdrucks hat. Als vegetativer Tonus wird dabei insbesondere der Vagustonus bzw. die Herzratenvariabilität und/oder ein Sympathikustonus und/oder ein vegetativer Quotient verwendet bzw. angesehen, wobei auch ein anderer durch das Stammhirn gesteuerter Tonus berücksichtigt werden kann. Vegetative Quotienten sind aus der AT 517 071 B1 der Anmelderin bekannt. Der vegetative Tonus hat direkten Einfluss auf den peripheren Widerstand der Blutgefäße und somit auch auf den tatsächlichen Blutdruck.

Mittels der gegenständlichen Messmethode kann der Blutdruck mittels weniger Messwerte bzw. innerhalb sehr weniger Herzschläge ermittelt werden. Dadurch kann auch die Herzschlag zu Herzschlag-Rhythmik des Blutdrucks gemessen werden. Es kann daher, im Gegensatz zur herkömmlichen Methode der Messungmittels Manschette ein Blutdruckverlauf zu den einzelnen Herzschlägen ermittelt werden, und entsprechend auch eine Blutdruckrhythmik.

Weiters kann dadurch, allein aus dem vegetativen Tonus bzw. der Pulswellengeschwindigkeit auch ein Wert für die Arterienelastizität ermittelt werden.

Die Erfindung betrifft weiters eine Vorrichtung gemäß Patentanspruch 8.

Aufgabe der Erfindung ist es daher eine Vorrichtung der vorstehend genannten Art anzugeben, mit welcher die genannten Nachteile vermieden werden können, mit welcher einfach eine genaue Ermittlung wenigstens eines Kreislaufparameters, insbesondere des Blutdrucks, eines Probanden möglich ist.

Erfindungsgemäß wird dies durch die Merkmale des Patentanspruches 8 erreicht. Dadurch können die vorstehend zum Verfahren geltend gemachten Vorteile erzielt werden.

Die Unteransprüche betreffen weitere vorteilhafte Ausgestaltungen der Erfindung. Ausdrücklich wird hiermit auf den Wortlaut der Patentansprüche Bezug genommen, wodurch die Ansprüche an dieser Stelle durch Bezugnahme in die Beschreibung eingefügt sind und als wörtlich wiedergegeben gelten.

Die Erfindung wird unter Bezugnahme auf die beigeschlossenen Zeichnungen, in welchen lediglich bevorzugte Ausführungsformen beispielhaft dargestellt sind, näher beschrieben. Dabei zeigt:

> Fig. 1 ein Blockschaltbild einer bevorzugten Ausführungsform eines gegenständlichen Verfahrens;
> Fig. 2 ein Blockschaltbild einer bevorzugten Ausführungsform einer gegenständlichen Vorrichtung;
> Fig. 3 eine bevorzugte Ausführungsform des Kontaktbereichs einer gegenständlichen Vorrichtung als Toilettensitz; und
> Fig. 4 eine bevorzugte Ausführungsform einer gegenständlichen Vorrichtung als Personenwaage.

Bei einem Verfahren zur Ermittlung wenigstens eines Blutdruckwertes eines Probanden ist vorgesehen, mittels einer Herzschlagmessanordnung 2 ein Herzschlagsignal des Probanden zu detektieren. Dabei kann es sich um jede Art einer entsprechenden Messanordnung handeln, wobei ein EKG-Gerät vorgesehen ist. Weiters kann zur Aufzeichnung des Herzschlagsignals (in nicht unter die Ansprüche fallenden Verfahren und Vorrichtungen) auch die Ballistokardiographie, die Ultraschallkardiographie, die Magnetokardiographie, die Sonokardiographie oder kapazitive Messungen verwendet werden, und die Herzschlagmessanordnung 2 auch entsprechend ausgebildet sein.

Das ermittelte Herzschlagsignal wird an eine Kreislaufparameterermittlungseinheit 4 übermittelt, welche bevorzugt umfassend einen Mikrocomputer bzw. Mikrokontroller ausgebildet ist. Die Kreislaufparameterermittlungseinheit 4 ermittelt aus dem zeitlichen Verlauf des Herzschlagsignals wenigstens einen Wert eines vegetativen Tonus. Dabei wird der Vagustonus bzw. in nicht unter die Ansprüche fallenden Beispielen die

Herzratenvariabilität und/oder der Sympathikustonus und/oder ein vegetativen Quotienten. Die Ermittlung erfolgt bei Auswertung eines EKG in an sich bekannter Weise über die Aufzeichnung der unterschiedlichen zeitlichen Dauer aufeinander folgender R-R Intervalle. R bezeichnet dabei in an sich bekannter Weise die R-Zacke. Diesbezüglich wird weiters auf die AT 515.102 und die WO 2015/176088 der Anmelderin verwiesen, in welchen die Messung des Vagustonus ausführlich beschrieben ist.

Die Kreislaufparameterermittlungseinheit 4 ermittelt in weiterer Folge einen Wert für einen Blutdruck des Probanden und gibt diesen Wert über eine Anzeige aus bzw. speichert oder versendet diesen Wert. Bevorzugt ist vorgesehen, dass der Wert für den Blutdruck fortlaufend ermittelt bzw. aktualisiert wird. Weiters ist bevorzugt vorgesehen, dass sowohl Werte für den systolischen und den diastolischen Blutdruck und/oder die Blutdruckamplitude ermittelt und ausgegeben werden. Bevorzugt ermittelt die Kreislaufparameterermittlungseinheit 4 weiters auch Werte für eine Arterienelastizität und/oder eine Pulswellengeschwindigkeit PWV und gibt diese aus und/oder speichert diese.

Bei der Ermittlung des Blutdruckwertes wird der wenigstens eine Wert des Vagustonus berücksichtigt. Nachfolgend wird eine erste bevorzugte Umsetzung des gegenständlichen Verfahrens beschrieben.

Die Kreislaufparameterermittlungseinheit (4) ermittelt aus dem vegetativen Tonus einen Zeitwert einer Preejection Period PEP. Über diese Pre-ejection Period PEP wird der Vagustonus bei der Ermittlung des wenigstens einen Blutdruckwerts berücksichtigt. Dabei hat es sich als Vorteilhaft erwiesen, wenn nicht irgendein Wert eines Vagustonus verwendet wird, sondern ein statistisch aussagekräftiger Wert.

Eine erste bevorzugte Gleichung für den Median der gemessenen Vagustonusse lautet:

$$VT = median\ von\ \{\log[absolut\ (RR_{n+1} - RR_n)]\}$$

Eine zweite bevorzugte Gleichung für den Mittelwert der gemessenen Vagustonusse lautet:

$$VT = log\left|\frac{1}{N}\sum_{i=1}^{N}[absolut(RR_{n+1} - RR_n)]\right|$$

Es hat sich als vorteilhaft erwiesen, wenn n größer gleich 4 ist.

Weiters hat es sich als vorteilhaft für die Genauigkeit des ermittelten Blutdruckwertes erwiesen, wenn diese Werte VT weiters korrigiert bzw. normiert werden.

Eine erste bevorzugte Gleichung für eine entsprechend korrigierten Vagustonus VT lautet:

$$VT_{corr} = \frac{median\ von\ \{\log[absolut\ (RR_{n+1} - RR_n)]\}}{G} * 100$$

Dabei wird der Median ermittelt, und weiters korrigiert. Eine zweite bevorzugte Gleichung für den entsprechend korrigierten Vagustonus VT lautet:

$$VT_{corr} = \frac{log \left| \frac{1}{N} \sum_{i=1}^{N} [absolut(RR_{n+1} - RR_n)] \right|}{G} * 100$$

Dabei wird anstelle des Median der Mittelwert gebildet und weiters korrigiert.

Der Koeffizient G bezeichnet dabei eine Korrekturvariable. Dabei ist vorgesehen, dass der Wert dieser Korrekturvariablen durch Vergleich der gemessenen Daten für den Vagustonus mit gespeicherten Daten ermittelt wird. Hiezu ist vorgesehen, dass in einer Datenbank entsprechende Messwerte für den Vagustonus angelegt werden, und zwar verknüpft mit möglichst vielen der folgenden Eigenschaften: Alter des Probanden, biologisches Geschlecht des Probanden, Armlänge des Probanden, Körpergröße des Probanden, Gewicht des Probanden, ethnische Herkunft, regionale Herkunft, Arzneimittelkonsum, Drogenkonsum, Wohnort, Ernährungsgewohnheiten, Schlaf/Wachgewohnheiten. Weiters hat es sich als besonders vorteilhaft erwiesen, wenn die aufgenommenen Daten zum Vagustonus, also die sog. Vagustonusvergleichswerte, noch hinsichtlich der Uhrzeit bzw. Tageszeit, sowie der Jahreszeit in der Datenbank abgelegt sind. Bei einem neuen Probanden werden dann anhand der entsprechenden Körpereigenschaften, der aktuellen Uhr- bzw. Tageszeit und Jahreszeit, sowie den weiteren Lebensumständen die Daten aus der Datenbank ausgelesen, bei denen die jeweiligen Probanden passende bzw. ähnliche Eigenschaften aufgewiesen haben. Weiters ist es vorteilhaft, wenn zudem jeweils die Daten verwendet werden, welche auch hinsichtlich der der Uhrzeit bzw. Tageszeit, sowie der Jahreszeit mit dem aktuellen Probanden übereinstimmen.

Da die gespeicherten Einträge ebenfalls einer Streuung unterliegen ist diesbezüglich bevorzugt vorgesehen, zu den gespeicherten Messwerten, welche hinsichtlich der Eigenschaften und/oder der Zeit passend sind und entsprechend ausgewählt wurden, für G einen Durchschnittswert zu ermitteln. Dieser Durchschnittswert kann etwa der arithmetische Mittelwert der ausgewählten Vagustonusvergleichswerte sein. Es hat sich als vorteilhafter erwiesen, wenn der Durchschnittswert ein Medianwert der ausgewählten Vagustonusvergleichswerte ist. Besonders bevorzugt ist vorgesehen, dass der Wert von G ein sog. Perzentilwert der ausgewählten Vagustonusvergleichswerte bezogen auf eine vorgebbare bzw. einzugebende Grenze in %, etwa ein 50% oder ein 90% Perzentilwert, ist.

G ist daher ein, aus gespeicherten Vagustonusvergleichswerten ermittelter Durchschnittswert eines Vagustonus.

Anhand des variablen Koeffizienten G kann der Vagustonus VT dahin gehend korrigiert werden, ob dieser höher bzw. niedriger ist als die gespeicherten Einträgen, angepasst an die jeweiligen, genannten Faktoren bzw. Eigenschaften. Der aktuelle Vagustinus wird daher auf den Wert G normiert. Durch die Multiplikation mit dem Wert 100 kann erreicht werden, dass der Ausgabewert ein Prozentwert von G ist. Der korrigierte VT ist dann $VT_{corr}$.

Es sei darauf hingewiesen, dass sich insbesondere der Abgleich der Vergleichsdaten mit den aktuellen Messdaten hinsichtlich der Uhr- bzw. Tageszeit als besonders effektiv erwiesen hat, um die Genauigkeit des gegenständlichen Verfahrens weiter zu erhöhen.

Entsprechend dem Vagustonus kann (in nicht unter die Ansprüche fallenden Beispielen) auch der Autonome Tonus AT bzw. der Sympathische Tonus ST bzw. die Herzratenvariabilität HRV verwendet werden. Dabei ist jeweils insbesondere vorgesehen, diese in der vorbeschriebenen Weise korrigiert werden.

Im Rahmen der gegenständlichen Anmeldung ist bevorzugt vorgesehen, zu sämtlichen ermittelten Werten einen entsprechen korrigierten Wert zu bilden, und mit diesem weiter zu arbeiten.

Aus dem VT kann die PEP auf unterschiedliche Weise ermittelt werden. Folgende bevorzugte Gleichung hat sich dabei als vorteilhaft erwiesen, wobei bereits der korrigierte $VT_{corr}$ verwendet wird. Alternativ kann auch ein anderer vegetativer Tonus verwendet werden. Dabei wird mittels des vegetativen Tonus ein Zeitwert einer Pre-ejection Period ermittelt. Weiters fließen dabei bevorzugt der zeitliche RR-Abstand bzw. die Herzschlagfrequenz zur Zeit der Messung in die Gleichung ein.

$$PEP_{Corr} = [0,03 * RR + 88,36] - \left\{ \frac{[(0,03 * RR + 88,3) - (0,021 * RR + 89,2)] * VT_{corr}}{100} \right\}$$

In der vorstehenden Gleichung kann alternativ auch ein korrigiertes Intervall $RR_{corr}$ eingetragen, welches dann an Stelle des gemessenen Intervalls bzw. der gemessenen Zeitspanne RR treten würde. $RR_{corr}$ würde dabei entsprechend $VT_{corr}$ ermittelt, wie dies bereits ausführlich erläutert wurde. Dabei werden aus gespeicherten Vergleichsdaten zu RR-Intervallen sowie der Eingabe der entsprechenden Eigenschaften und/oder der Zeit der Messung, passende Vergleichsdaten ermittelt, wie dies ausführlich zum Koeffizienten G beschrieben ist.

Es wird mittels einer Pulsmessanordnung an einem peripheren Blutgefäß ein Pulssignal des Probanden ermittelt und an die Kreislaufparameterermittlungseinheit 4 übermittelt. Besonders bevorzugt ist dabei vorgesehen, dass die Kreislaufparameterermittlungseinheit 4 auch einen ermittelten Verlauf des Pulssignals, insbesondere eine Pulsweite eines Pulses innerhalb des Pulssignals, bei der Ermittlung des wenigstens einen Blutdruckwerts berücksichtigt.

Aus dem Herzschlagsignal und dem Pulssignal am peripheren Blutgefäß wird eine Puls-Arrival Time PAT ermittelt. Die Puls-Arrival Time ist die Zeit, welche zwischen einem ersten Herzschlag und dem Auftreten eines zugehörigen ersten Pulssignals am peripheren Blutgefäß vergeht. Dabei können unterschiedliche Arten der Bestimmung dieser Puls-Arrival Time bzw. PAT vorgesehen sein. Beim Herzschlagsignal wird jeweils von der R-zacke weg gemessen, und zwar von deren Auftreten weg entweder bis zum Beginn der zugehörigen Pulswelle, oder bis zur größten Steigung der betreffenden Pulswelle oder bis zum Scheitelpunkt der betreffenden Pulswelle. Die sog. PAT wird daher direkt aus zwei unmittelbaren Messwerten ermittelt. Weiters wird bevorzugt ein Durchschnittswert für die PAT ermittelt.

Bevorzugt ist weiters vorgesehen, dass neben der PAT auch eine $PAT_{corr}$ gebildet wird, und zwar analog der beschriebenen Ermittlung der $VT_{corr}$. Es wird daher aus gespeicherten Vergleichsdaten ein Koeffizient gebildet, auf welchen die PAT normiert wird.

In weiterer Folge wird eine korrigierte Pulslaufzeit PTT ermittelt, indem die Pre-ejection Period von der Puls-Arrival Time abgezogen wird. PTT steht dabei für Puls Transit Time. Dabei hat sich insbesondere folgende Formel als vorteilhaft erwiesen:

$$PTT = \left\{ \frac{1}{N} * \sum_{i=1}^{N} PAT_i \right\} - PEP_{Corr}$$

Alternativ kann anstatt dem Mittelwert in der vorstehenden Gleichung auch der Median PAT verwendet werden. Die PTT kann weiters korrigiert werden.

Weiters wird ein Wert für eine arterielle Länge $l_{art}$ zu dem peripheren Blutgefäß des Probanden, insbesondere durch Abmessen der entsprechenden Länge, ermittelt, und an die Kreislaufparameterermittlungseinheit 4 übermittelt wird. Die betreffende Länge kann etwa aufgrund der äußeren Abmessungen des Probanden ermittelt werden. Insbesondere ist die arterielle Länge $l_{art}$ die Entfernung von der Ventilebene des Herzens zu der Pulsmessstelle am Blutgefäß des Probanden, wobei die entlang dem betreffenden Blutgefäß erfolgt.

Bevorzugt wird die die arterielle Länge bei der Ermittlung des wenigstens einen Blutdruckwerts berücksichtigt.

Die Kreislaufparameterermittlungseinheit 4 ermittelt aus der arteriellen Länge und der korrigierten Pulslaufzeit eine Pulswellengeschwindigkeit PWV. Dabei gilt, wie an sich bekannt:

$$v = \frac{s}{t}$$

Entsprechend gilt:

$$PWV = \frac{l_{art}}{PTT}$$

bzw. :

$$PWV_{raw} = \frac{l_{art}}{PAT}$$

Weiters ist bevorzugt vorgesehen, dass die Kreislaufparameterermittlungseinheit 4 den Blutdruckwert aus wenigstens einer Gleichung ermittelt, welche Gleichung wenigstens die Pulswellengeschwindigkeit als Variable sowie eine Mehrzahl an Koeffizienten aufweist. Die betreffende Gleichung kann dabei ein lineares Gleichungssystem sein, bzw. weiters auch quadratische oder kubische Terme sowie Konstante aufweisen. Nachfolgend wird ein erstes Beispiel für entsprechende Gleichungen für den systolischen Blutdruck $BP_{sys}$ und den diastolischen Blutdruck $BP_{dia}$ angegeben:

$$BP_{sys} = S - T * (PWV \; oder \; PWV_{raw}) + H * (PWV \; oder \; PWV_{raw})^2$$

$$BP_{dia} = BP_{sys} - \{U - V * (PWV \; oder \; PWV_{raw}) + W * (PWV \; oder \; PWV_{raw})^2 \}$$

Die betreffenden Koeffizienten S, T, H, U, V und W können auf unterschiedliche Weise ermittelt werden.

Gemäß einer ersten bevorzugten Variante ist vorgesehen, dass in einem Kalibrierungsschritt an dem Probanden ein Referenzblutdruck direkt gemessen und an die Kreislaufparameterermittlungseinheit 4 übermittelt werden, und dass die Kreislaufparameterermittlungseinheit 4 durch Vergleich des gemessenen Referenzblutdrucks und der Pulswellengeschwindigkeit Werte für die Mehrzahl an Koeffizienten ermittelt. Diese Referenzmessung kann etwa in an sich bekannter Weise mittels einer Blutdruckmessmanschette ermittelt werden. Die Kreislaufparameterermittlungseinheit 4 variiert dann Werte für die betreffenden Koeffizienten bis passende Werte ermittelt werden. Gleichzeitig damit kann auch errechnet werden, wie genau die Annäherung an die Messwerte gelingt.

Die Kalibrierung kann verbessert werden, wenn hiezu eine Mehrzahl an Messungen durchgeführt wird, etwa zu unterschiedlichen Tageszeiten bzw. in unterschiedlichen Situationen bzw. Belastungszuständen. Aufgrund dieser Kalibrierung kann dann eine Zuordnung bestimmter Datensätze einer Vielzahl gespeicherter Datensätze zu dem Probanden erfolgen Gemäß einer zweiten bevorzugten Variante ist vorgesehen, dass wenigstens ein Wert, insbesondere eine Wertegruppe, betreffend wenigstens eine vorgebbare Körpereigenschaft und/oder Lebensgewohnheit des Probanden an die Kreislaufparameterermittlungseinheit 4 übermittelt wird, und dass die Kreislaufparameterermittlungseinheit 4 die Mehrzahl an Koeffizienten ermittelt indem die eingegebenen Werte mit gespeicherten Vergleichsdatensätzen verglichen werden, wobei aufgrund eines vorgebbaren Maßes an Übereinstimmung und/oder Ähnlichkeit der eingegebenen Werte mit den gespeicherten Vergleichsdatensätzen Koeffizienten ausgewählt werden. Diese Variante kommt ohne eine Referenzmessung aus. Allerdings ist es dafür erforderlich zuvor an einer repräsentativen Menge bzw. Gruppe Blutdruckwerte zu messen und diese zusammen mit Werten, insbesondere eine Wertegruppe, betreffend wenigstens eine vorgebbare Körpereigenschaft und/oder Lebensgewohnheit des jeweils gemessenen Probanden aufzunehmen, und derart die Vergleichsdatensätze zu bilden. Dies stellt keine Schwierigkeit für den Fachmann dar, da lediglich eine entsprechende Menge an Messungen durchgeführt werden muss.

Bevorzugt ist die wenigstens eine Körpereigenschaft des Probanden wenigstens eine Körpereigenschaft ausgewählt aus der Gruppe: Alter des Probanden, biologisches Geschlecht des Probanden, Armlänge des Probanden, Körpergröße des Probanden, Gewicht des Probanden, ethnische Herkunft, regionale Herkunft.

Bevorzugt ist die wenigstens eine Lebensgewohnheit des Probanden eine Lebensgewohnheit ausgewählt aus der Gruppe: Arzneimittelkonsum, Drogenkonsum, Wohnort, Ernährungsgewohnheiten, Schlaf/Wachgewohnheiten,

Der nächste Proband muss lediglich die entsprechenden Angaben eingeben bzw. an die Kreislaufparameterermittlungseinheit 4 übermitteln, welche dann anhand der besten Übereinstimmungen Werte für die Koeffizienten auswählt.

Insbesondere ist vorgesehen, dass bei der Ermittlung der betreffenden Koeffizienten weiters die Tages- bzw. Uhrzeit sowie weiters die Jahreszeit berücksichtigt wird, da dadurch die Genauigkeit des gegenständlichen Verfahrens weiter gesteigert werden kann.

Das Bestimmen bzw. Auswählen der Koeffizienten ist mit Mitteln der computerimplementierten Mathematik einfach möglich. Die Zuordnung kann mittels Software, etwa zufolge der kleinsten Fehlerquadrate, oder einem neuronalen Netz oder Methoden der künstlichen Intelligenz erfolgen.

Alternativ zur vorstehend beschriebenen ersten bevorzugten Umsetzung des gegenständlichen Verfahrens wird nun eine zweite bevorzugte Umsetzung des gegenständlichen Verfahrens beschrieben. Diese zweite Umsetzung ist dabei offener bzw. weniger spezifiziert als die erste bevorzugte Umsetzung.

Die Kreislaufparameterermittlungseinheit 4 ermittelt dabei den systolischen Blutdruck zufolge folgendem bevorzugtem Zusammenhang, wobei jedoch einzelne Terme dieser Gleichung auch entfallen können:

$$P_{sys} = a*PWV + b*AT + c*HR + d*EBT + e*PW + f$$

Die Kreislaufparameterermittlungseinheit 4 ermittelt dabei den diastolischen Blutdruck zufolge folgendem bevorzugtem Zusammenhang:

$$P_{dia} = m*PWV + n*AT + o*HR + p*EBT + q*PW + r$$

Es kann auch vorgesehen sein, zu den einzelnen Faktoren dieser Gleichungen Terme höherer Ordnung, als etwa $PWV^2$ bzw. $PWV^3$ zu verwenden. Es hat sich gezeigt, dass die Berechnung dieser und auch der weiteren gegenständlich beschriebenen Werte mittels quadratischer bzw. kubischer Terme für manche Gruppen bessere Ergebnisse zur Folge haben kann.

Dabei bezeichnet AT den "autonomic nervous system tone" oder auch ANS tone. Dabeikann es sich um einen VT bzw. einen ST handeln.

HR steht für Heart Rate oder Herzrate.

EBT bezeichnet die Extremity Body Temperatur, also die Temperatur einer Extremität des Probanden. Es ist daher gemäß einer besonders bevorzugten Ausführungsform vorgesehen, dass mittels einer Temperaturmessanordnung eine Körpertemperatur an einem peripheren Körperteil des Probanden aufgenommen und an die Kreislaufparameterermittlungseinheit 4 übermittelt wird, und dass die Kreislaufparameterermittlungseinheit 4 eine ermittelte periphere Körpertemperatur bei der Ermittlung des wenigstens einen Blutdruckwerts berücksichtigt.

PW steht für die sog. "Pulse width", also die Pulsweite eines optional zu ermittelten peripheren Pulssignals. Es ist daher gemäß einer besonders bevorzugten Ausführungsform vorgesehen, dass mittels einer Pulsmessanordnung 3 an einem peripheren Blutgefäß ein Pulssignal des Probanden ermittelt und an die Kreislaufparameterermittlungseinheit 4 übermittelt wird, und dass die Kreislaufparameterermittlungseinheit 4 einen ermittelten Verlauf des Pulssignals, insbesondere eine Pulsweite eines Pulses innerhalb des Pulssignals, bei der Ermittlung des wenigstens einen Blutdruckwerts berücksichtigt.

PWV bezeichnet die bereits beschriebene "Pulse wave velocity", also die Pulswellengeschwindigkeit.

Die Gleichungen der zweiten Umsetzung zeigen dabei, wie weitere Parameter bzw. Variable in die Berechnung des Blutdruckes einfließen können.

Die in den vorgenannten Beziehungen angeführten Koeffizienten können mittels Versuchen bzw. einer Kalibrierung ermittelt werden, wie dies bereits zur ersten Umsetzung beschrieben ist.

Weiters ist bevorzugt vorgesehen, dass wenigstens ein Wert für einen elektrischen Hautwiderstand, eine Hauttemperatur, eine Gewebeleitfähigkeit, eine Gewebekapazität ermittelt wird und bei der Ermittlung des wenigstens einen Blutdruckwerts berücksichtigt wird. Da sich gezeigt hat, dass auch diese Faktoren Einfluss auf den Blutdruck bzw. einen vegetativen Tonus, etwa den Vagustonusn oder Sympatikustonus, bzw. eine vegetative Rhythmusamplitude, etwa einen Vagusrhythmus oder einen Sympatikustonus, haben können, kann durch deren Berücksichtigung die Messung weiter verbessert werden.

Weitere Faktoren, welche gegenständlich berücksichtigt werden können sind der Puls/Atemquotient, die Atemrhythmik sowie die Puls-Atemkopplung. Für die Berechnung des Puls/Atemquotienten wird die Herzfrequenz, zum Beispiel aus dem EKG, herangezogen. Die für die Berechnung notwendige Atemfrequenz wird z.B. aus der Modulation der Herzschlagfrequenz durch Atmung, also die sog. respiratorische Sinusarhythmie, berechnet.

Bevorzugt ist vorgesehen, dass sämtliche Parameter mittels Vergleichsdaten sowie unter Bezugnahme auf Uhrzeit, Tageszeit bzw. Jahreszeit korrigiert werden, wie dies anhand des VT ausführlich beschrieben ist.

Bei dem weiters beschriebenen Verfahren zur Ermittlung wenigstens eines Kreislaufparameters eines Probanden ist ebenfalls vorgesehen, dass mittels einer Herzschlagmessanordnung 2 ein Herzschlagsignal, insbesondere ein EKG oder eines der weiteren vorgenannten Verfahren, eines Probanden ermittelt und an eine Kreislaufparameterermittlungseinheit 4 übermittelt wird. Die Kreislaufparameterermittlungseinheit 4 ermittelt aus den Verläufen des Herzschlagsignals wenigstens einen ersten Kreislaufparameterzwischenwert.

Sofern nicht anders ausgeführt entsprechen die gleich bezeichneten Komponenten bzw. Variablen der beiden beschriebenen Verfahren den jeweils selben Komponenten bzw. Variablen. Bei der Auslegung der Begriffe sind daher auch die Ausführungen zum jeweils anderen Verfahren heranzuziehen.

Bevorzugt handelt es sich bei den gegenständlich zu ermittelten Kreislaufparametern um Werte wenigstens eines vegetativen Tonus. Da aus diesen Werten ebenfalls beansprucht ist wenigstens einen Blutdruckwert des Probanden zu ermitteln, ist eine Kombination einzelner oder sämtlicher Verfahrensschritte der beiden Verfahren vorgesehen.

Mittels des gegenständlichen Verfahrens können sehr genaue Kreislaufparameterwerte ermittelt werden.

Es ist vorgesehen, dass wenigstens ein Wert, insbesondere eine Wertegruppe, betreffend wenigstens eine vorgebbare Körpereigenschaft und/oder Lebensgewohnheit des Probanden an die Kreislaufparameterermittlungseinheit 4 übermittelt wird. Dies erfolgt durch Eingabe der entsprechenden Angaben über ein Interface.

Bevorzugt ist vorgesehen, dass die wenigstens eine Körpereigenschaft des Probanden wenigstens eine Körpereigenschaft ausgewählt aus der Gruppe: Alter des Probanden, biologisches Geschlecht des Probanden, Armlänge des Probanden, Körpergröße des Probanden, Gewicht des Probanden, ethnische Herkunft, regionale Herkunft, ist. Weiters ist bevorzugt vorgesehen, dass die wenigstens eine Lebensgewohnheit des Probanden eine Lebensgewohnheit ausgewählt aus der Gruppe: Arzneimittelkonsum, Drogenkonsum, Wohnort, Ernährungsgewohnheiten, Schlaf/Wachgewohnheiten, ist. All diese Faktoren können sich auf die Ermittlung des Kreislaufparameterwertes auswirken, müssen dies aber nicht.

Als weiterer Wert ist bevorzugt vorgesehen, dass bei der Ermittlung des Kreislaufparameterrechenwerts eine aktuelle Uhrzeit oder Tageszeit oder Jahreszeit berücksichtigt wird. Dabei ist vorgesehen, dass in den Vergleichsdatensätzen auch entsprechend zu unterschiedlichen Uhrzeiten, Tageszeiten und/oder Jahreszeiten korrespondierende Datensätze abgelegt sind. Es hat sich gezeigt, dass ein und derselbe Messwert an einem Probanden eine andere Aussage hat, je nachdem zu welcher Tageszeit dieser ermittelt wird. So kann etwa ein und derselbe Blutdruckwert Mittags als unbedenklich eingestuft werden, am Abend aber als problematisch oder gar gefährlich.

Die Speicherung von tages- und/oder jahreszeitabhängigen Referenzdaten ist deshalb vorteilhaft, da sich die Zusammenhänge zwischen den Parametern und damit die Koeffizienten im Laufe des Tages bzw. Jahres ändern können und dadurch eine zeitabhängige Korrektur vorgenommen werden kann.

Aus dem wenigstens einen eingegebenen Wert, insbesondere der Wertegruppe, ermittelt die Kreislaufparameterermittlungseinheit 4 einen Kreislaufparameterrechenwert.

Bevorzugt ist vorgesehen, dass bei der Ermittlung des Kreislaufparameterrechenwerts jeder der eingegebenen Werte für Körpereigenschaft und/oder Lebensgewohnheit mit einem der jeweiligen Körpereigenschaft und/oder Lebensgewohnheit zugeordneten Koeffizienten multipliziert wird.

Dabei erfolgt die Ermittlung des Kreislaufparameterrechenwerts unter Berücksichtigung einer Vielzahl von Vergleichsdatensätzen, welche aus Referenzmessungen erstellt werden bzw. zuvor erstellt wurden. Dabei ist bevorzugt vorgesehen, dass die Koeffizienten zu den einzelnen Körpereigenschaften und/oder Lebensgewohnheiten durch Vergleich der eingegebenen Werte mit den in der Vielzahl von Vergleichsdatensätzen gespeicherten Werten und/oder Zusammenhängen ermittelt werden. Besonders bevorzugt ist dabei vorgesehen, dass die Koeffizienten zu den einzelnen Körpereigenschaften und/oder Lebensgewohnheiten mittels eines neuronalen Netzes aus der Vielzahl von Vergleichsdatensätzen ermittelt werden.

Es ist vorgesehen, dass die Kreislaufparameterermittlungseinheit 4 aus dem Kreislaufparameterzwischenwert, dem Kreislaufparameterrechenwert sowie einer vorgebbaren Streuungsbreite einen Kreislaufparameterausgabewert ermittelt, und diesen Kreislaufparameterausgabewert ausgibt.

In weiterer Folge kann aus dem Kreislaufparameterausgabewert jeweils ein Wert für den systolischen Blutdruck und den diastolischen Blutdruck ermittelt und ausgegeben werden, wobei die entsprechend zuvor beschriebenen Schritte für die Ermittlung eines Blutdruckwertes anzuwenden sind, wobei der vegetativen Tonus entsprechend dem gegenständlichen Verfahren ermittelt wird.

Bei der Ermittlung des Blutdrucks ist weiters bevorzugt vorgesehen, dass mittels einer Pulsmessanordnung 3 an einem peripheren Blutgefäß ein Pulssignal des Probanden ermittelt und an die Kreislaufparameterermittlungseinheit 4 übermittelt wird, und dass die Kreislaufparameterermittlungseinheit 4 ermittelte Verläufe des peripheren Pulssignals bei der Ermittlung des ersten Kreislaufparameterzwischenwerts berücksichtigt.

Weiters kann vorgesehen sein, auf diesem Wege die Vitalität eines Menschen zu überwachen bzw. aufgrund eines Blutdruckwertes oder eines Wertes eines anderen Kreislaufparameters eine Maschine zu steuern oder eine Handlung auszulösen. Dabei ist bevorzugt vorgesehen, dass der ermittelte Blutdruck mit wenigstens einem Vergleichskriterium verglichen wird, und dass bei Erfüllen des wenigstens einen Vergleichskriteriums ein Steuersignal bzw. ein Warnsignal ausgegeben wird.

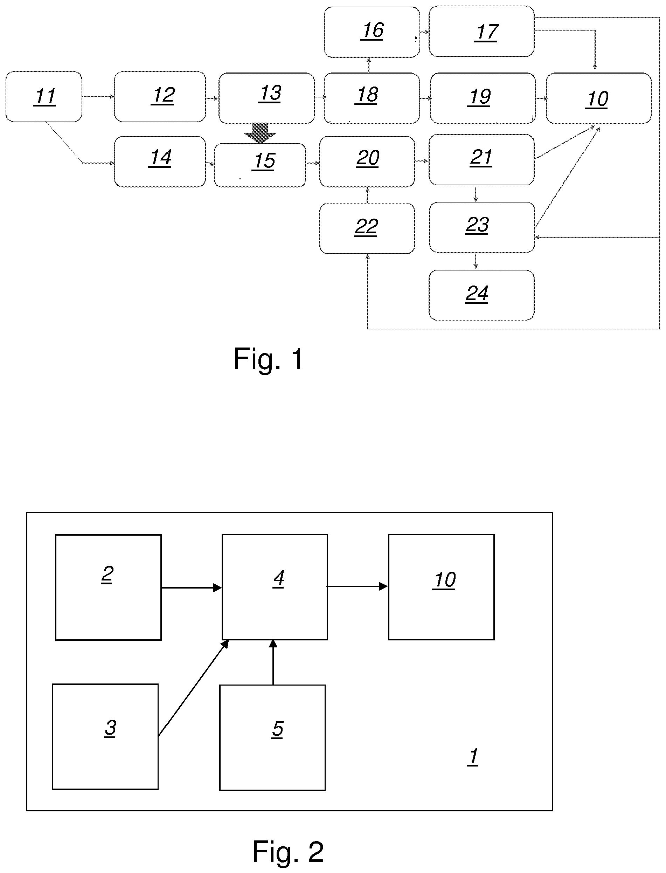

Die Fig. 1 zeigt ein Blockschaltbild eines bevorzugten Verfahrensablaufes zur Ermittlung eines Kreislaufparameters, insbesondere eines Blutdrucks eines Probanden. Dabei bezeichnet Bezugszeichen 11 den menschlichen oder tierischen Probanden. Selbstverständlich ist das gegenständliche Verfahren auf Tiere mit einem Kreislaufsystem eingeschränkt.

Bezugszeichen 12 bezeichnet die Aufnahme des Herzschlagsignals und Bezugszeichen 13 die Detektion de R-Zacke innerhalb des Herzschlagsignals. In Block 18 wird die Zeit zwischen zwei R Zacken bestimmt, und in Block 16 der AT ermittelt. In Block 19 wird die Herzrate ermittelt. In Block 17 erfolgt der optionale aber bevorzugte Abgleich mit den gespeicherten Vergleichsdatensätzen.

Bezugszeichen 10 bezeichnet ein Display.

Bezugszeichen 14 bezeichnet die Aufnahme der Pulswelle an dem peripheren Gefäß. Bei Bezugszeichen 15 wird ausgehend von einem Erwarteten Auftretenszeitpunkt ausgehend von der detektierten R-Zacke ein Zeitfenster über das Pulswellensignal gelegt. Bezugszeichen 20 bezeichnet die Detektion der Pulswelle innerhalb des überwachten Zeitfensters des Pulswellensignals.

In Block 21 erfolgt die Ermittlung der PWV. In Block 23 erfolgt die Ermittlung der Blutdruckwerte. Diese Ermittlung kann mittels der ermittelten AT-Werte erfolgen.

Bezugszeichen 22 bezeichnet die Korrektur des PEP.

Gemäß Block 24 ist die Steuerung einer Maschine vorgesehen.

Gemäß einem weiteren bevorzugten Verfahren kann vorgesehen sein, dass ein Reiz und/oder eine Atemauforderung in Abhängigkeit eines Verlaufes des Blutdrucks und/oder des Kreislaufparameterausgabewerts erzeugt und ausgegeben wird. Dadurch kann der Blutdruckverlauf für Biofeedback verwendet werden.

Bevorzugt ist dabei vorgesehen, dass eine Veränderung des Blutdrucks und/oder des Kreislaufparameterausgabewerts in Reaktion auf den ausgegebenen Reiz bzw. die Atemaufforderung ermittelt wird, und dass die Veränderung bei der Bestimmung nachfolgender Zeitpunkte zur Ausgabe des Reizes bzw. der Atemaufforderung berücksichtigt wird. Es hat sich dabei gezeigt, dass durch die gegenständlich beschriebene Vorgabe von Reizen bzw. Atemaufforderungen eine gezielte Beeinflussung des Blutdruckverlaufes eines Probanden erreicht werden kann. Dadurch kann gezielt ein bestimmter Zustand eines Probanden hervorgerufen werden, welcher sich sowohl physisch wie auch psychisch auf diesen auswirkt. Dadurch kann das Wohlbefinden und die Leistungsfähigkeit eines Probanden nachhaltig und einfach gesteigert werden. Dadurch kann der Gesundheitszustand eines Probanden verbessert werden.

Es ist weiters eine Vorrichtung 1 zur Ermittlung wenigstens eines Kreislaufparameters eines Probanden vorgesehen, wobei die Vorrichtung eine Herzschlagmessanordnung 2, eine Pulsmessanordnung 3 und eine Kreislaufparameterermittlungseinheit 4 aufweist, wobei die Vorrichtung 1 einen Datenspeicher 5 mit einer Vielzahl von Vergleichsdatensätzen, umfassend Zusammenhänge zwischen Werten für Körpereigenschaften und/oder Lebensgewohnheiten des Probanden und Kreislaufparameterwerten des Probanden, aufweist, und dass die Kreislaufparameterermittlungseinheit 4 zur Durchführung eines gegenständlichen Verfahrens ausgebildet ist. Fig. 2 zeigt ein Blockschaltbild einer entsprechenden Vorrichtung.

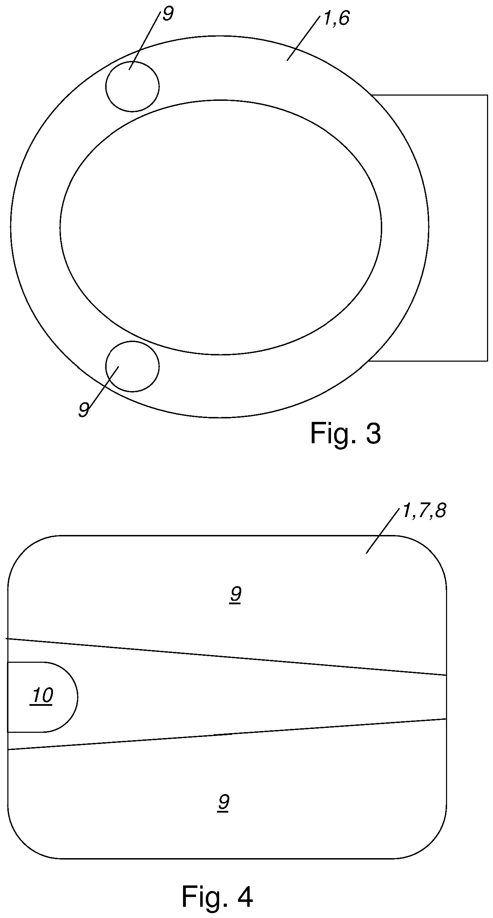

Gemäß einer ersten bevorzugten Ausführungsform ist vorgesehen, dass zumindest Kontaktbereiche 9 der Herzschlagmessanordnung 2 und/oder der Pulsmessanordnung 3 in einer Sitzfläche, insbesondere einer Toilettensitzfläche 6, angeordnet sind. Fig. 3 zeigt eine schematische Darstellung eines entsprechenden Toilettensitzes. Als weitere bevorzugte Ausführungsformen sind vorgesehen, dass die Sitzfläche Teil einer Badewanne bzw. eines Arbeits- bzw. Bürostuhls ist. Ebenso kann eine Liegefläche, insbesondere eine Therapieliege oder ein Bett entsprechend ausgebildet sein.

Gemäß einer zweiten bevorzugten Ausführungsform ist vorgesehen, dass zumindest Kontaktbereiche 9 der Herzschlagmessanordnung 2 und/oder der Pulsmessanordnung 3 in einer Standfläche 7, insbesondere einer Personenwaagenstandfläche 8, angeordnet sind. Fig. 4 zeigt eine schematische Darstellung einer entsprechenden Personenwaage. Als weitere bevorzugte Ausführungsformen sind vorgesehen, dass die Standfläche als Teil einer Duschtasse ausgebildet.

Gemäß einer dritten bevorzugten Ausführungsform ist vorgesehen, dass zumindest Kontaktbereiche 9 der Herzschlagmessanordnung 2 und/oder der Pulsmessanordnung 3 in einem Steuer- und/oder Bedienelement, insbesondere einem Lenkrad, einem Zweiradlenker, einem Steuerrad und/oder einem Steuerknüppel, einer Maschine, insbesondere einem Land- und/oder Luft- und/oder Wasserfahrzeug, angeordnet sind.

Bei den gegenständlich beschriebenen Verfahren ist es nicht zwingend erforderlich, das immer sämtlich ermöglichen bzw. auch nur sämtliche verfügbaren Parameter in die Ermittlung bzw. Berechnung eines Wertes einfließen. Es hat sich als besonders vorteilhaft erwiesen, bestimmte Werte mehrfach unter Anwendung jeweils unterschiedlicher Parameter bzw. Parameterkombination zu ermitteln, sowie jeweils eine Fehlerwahrscheinlichkeit zu diesen Ermittlungen zu erstellen, und danach aus einem Vergleich bzw. einem Zusammenführen der unterschiedlich ermittelten Werte einen endgültigen Ausgabewert zu bestimmen.

## Patentansprüche

1. Verfahren zur Ermittlung wenigstens eines Blutdruckwertes eines Probanden,

- wobei mittels eines EKG-Geräts (2) ein EKG eines Probanden ermittelt und an eine Kreislaufparameterermittlungseinheit (4) übermittelt wird,
- wobei die Kreislaufparameterermittlungseinheit (4) aus einem ermittelten zeitlichen Verlauf des EKGs wenigstens einen Wert eines Vagustonus ermittelt,
- wobei durch die Kreislaufparameterermittlungseinheit (4) mittels des wenigstens einen Werts des Vagustonus ein Zeitwert einer Pre-ejection Period ermittelt wird,
- wobei mittels einer Pulsmessanordnung an einem peripheren Blutgefäß ein Pulssignal des Probanden ermittelt und an die Kreislaufparameterermittlungseinheit (4) übermittelt wird,
- wobei aus dem EKG und dem Pulssignal am peripheren Blutgefäß eine Puls-Arrival Time ermittelt wird, wobei die Puls-Arrival Time die Zeit ist, welche zwischen einem ersten Herzschlag und dem Auftreten eines zugehörigen ersten Pulssignals am peripheren Blutgefäß vergeht,
- wobei eine korrigierte Pulslaufzeit ermittelt wird, indem die Pre-ejection Period von der Puls-Arrival Time abgezogen wird,
- wobei ein Wert für eine arterielle Länge zu dem peripheren Blutgefäß des Probanden ermittelt, und an die Kreislaufparameterermittlungseinheit (4) übermittelt wird,
- wobei durch die Kreislaufparameterermittlungseinheit (4) aus der arteriellen Länge und der korrigierten Pulslaufzeit eine Pulswellengeschwindigkeit ermittelt wird,
- wobei die Kreislaufparameterermittlungseinheit (4) den wenigstens einen Blutdruckwert aus wenigstens einer Gleichung ermittelt, welche Gleichung wenigstens die Pulswellengeschwindigkeit als Variable sowie eine Mehrzahl an Koeffizienten aufweist,

- wobei in einem Kalibrierungsschritt an dem Probanden ein Referenzblutdruck direkt gemessen und an die Kreislaufparameterermittlungseinheit (4) übermittelt wird, und wobei die Kreislaufparameterermittlungseinheit (4) durch Vergleich des gemessenen Referenzblutdrucks und der Pulswellengeschwindigkeit Werte für die Mehrzahl an Koeffizienten ermittelt,

oder

wobei eine Wertegruppe betreffend wenigstens eine vorgebbare Körpereigenschaft und/oder Lebensgewohnheit des Probanden an die Kreislaufparameterermittlungseinheit (4) übermittelt wird, und wobei die Kreislaufparameterermittlungseinheit (4) die Mehrzahl an Koeffizienten ermittelt indem die eingegebenen Werte mit gespeicherten Vergleichsdatensätzen verglichen werden, wobei aufgrund eines vorgebbaren Maßes an Übereinstimmung und/oder Ähnlichkeit der eingegebenen Werte mit den gespeicherten Vergleichsdatensätzen die Koeffizienten ausgewählt werden,

- wobei der ermittelte wenigstes eine Blutdruckwert ausgegeben wird.

2. Verfahren nach Anspruch 1, **dadurch gekennzeichnet, dass** der Wert des vegetativen Tonus, vor dessen Berücksichtigung bei der Ermittlung des Blutdruckwertes, anhand gespeicherter Vergleichsdatensätze normiert wird.

3. Verfahren nach Anspruch 1 oder 2, **dadurch gekennzeichnet, dass** der Wert für die arterielle Länge zu dem peripheren Blutdruckgefäß des Probanden durch Abmessen der entsprechenden Länge ermittelt wird.

4. Verfahren nach einem der Ansprüche 1 bis 3, **dadurch gekennzeichnet, dass** die wenigstens eine Körpereigenschaft des Probanden wenigstens eine Körpereigenschaft ausgewählt aus der Gruppe: Alter des Probanden, biologisches Geschlecht des Probanden, Armlänge des Probanden, Körpergröße des Probanden, Gewicht des Probanden, ethnische Herkunft, regionale Herkunft, ist.

5. Verfahren nach einem der Ansprüche 1 bis 4, **dadurch gekennzeichnet, dass** die wenigstens eine Lebensgewohnheit des Probanden eine Lebensgewohnheit ausgewählt aus der Gruppe: Arzneimittelkonsum, Drogenkonsum, Wohnort, Ernährungsgewohnheiten, Schlaf/Wachgewohnheiten, ist.

6. Verfahren nach einem der Ansprüche 1 bis 5, **dadurch gekennzeichnet, dass** bei der Ermittlung des Blutdruckwertes eine aktuelle Uhrzeit oder Tageszeit oder Jahreszeit berücksichtigt wird, insbesondere dass zu den Vergleichsdatendatensätzen jeweils die Uhrzeit oder Tageszeit oder Jahreszeit gespeichert ist, zu denen diese erstellt wurden.

7. Verfahren nach einem der Ansprüche 1 bis 6, **dadurch gekennzeichnet, dass** jeweils ein Wert für den systolischen Blutdruck und/oder eine Blutdruckamplitude und den diastolischen Blutdruck und/oder eine Blutdruckamplitude ermittelt und ausgegeben wird.

8. Vorrichtung (1) zur Ermittlung wenigstens eines Kreislaufparameters eines Probanden, wobei die Vorrichtung eine Herzschlagmessanordnung (2), eine Pulsmessanordnung (3) und eine Kreislaufparameterermittlungseinheit (4) aufweist, **dadurch gekennzeichnet, dass** die Vorrichtung (1) zur Durchführung eines Verfahrens nach einem der Ansprüche 1 bis 7 ausgebildet ist.

**Claims**

1. Method for determining at least one blood pressure value of a test person,

- wherein an ECG of a subject is determined by means of an ECG device (2) and transmitted to a circulation parameter determination unit (4),
- wherein the circulation parameter determination unit (4) determines at least one value of a vagal tone from a determined time course of the ECG,
- wherein a time value of a pre-ejection period is determined by the circulation parameter determination unit (4) by means of the at least one value of the vagal tone,
- wherein a pulse signal of the subject is determined by means of a pulse measuring arrangement on a peripheral blood vessel and transmitted to the circulation parameter determination unit (4),
- wherein a pulse arrival time is determined from the ECG and the pulse signal at the peripheral blood vessel, the pulse arrival time being the time which elapses between a first heartbeat and the occurrence of an associated first pulse signal at the peripheral blood vessel,
- wherein a corrected pulse transit time is determined by subtracting the pre-ejection period from the pulse arrival time,
- wherein a value for an arterial length to the peripheral blood vessel of the subject is determined and transmitted to the circulation parameter determination unit (4),
- wherein a pulse wave velocity is determined by the circulation parameter determination unit (4) from the arterial length and the corrected pulse transit time,
- wherein the circulation parameter determination unit (4) determines the at least one blood pressure value from at least one equation, which equation comprises at least the pulse wave velocity as a variable and a plurality of coefficients,

- wherein in a calibration step a reference blood pressure is measured directly on the subject and transmitted to the circulation parameter determination unit (4), and wherein the circulation parameter determination unit (4) determines values for the plurality of coefficients by comparing the measured reference blood pressure and the pulse wave velocity,
or
wherein a group of values relating to at least one predeterminable body characteristic and/or lifestyle habit of the subject is transmitted to the circulation parameter determination unit (4), and wherein the circulation parameter determination unit (4) determines the plurality of coefficients by comparing the input values with stored comparison data sets, wherein the coefficients are selected on the basis of a predeterminable degree of agreement and/or similarity of the input values with the stored comparison data sets,

- whereby the determined at least one blood pressure value is output.

2. Method according to claim 1, **characterized in that** the value of the vegetative tone is normalized on the basis of stored comparative data sets before it is taken into account in determining the blood pressure value.

3. Method according to claim 1 or 2, **characterized in that** the value for the arterial length to the peripheral blood pressure vessel of the subject is determined by measuring the corresponding length.

4. Method according to one of claims 1 to 3, **characterized in that** the at least one body characteristic of the subject is at least one body characteristic selected from the group: age of the subject, biological sex of the subject, arm length of the subject, height of the subject, weight of the subject, ethnic origin, regional origin.

5. Method according to any one of claims 1 to 4, **characterized in that** the at least one lifestyle habit of the subject is a lifestyle habit selected from the group: drug consumption, drug use, place of residence, eating habits, sleeping/waking habits.

6. Method according to one of claims 1 to 5, **characterized in that** a current time of day or time of day or time of year is taken into account when determining the blood pressure value, in particular **in that** the time of day or time of day or time of year at which the comparison data sets were created is stored for each of the comparison data sets.

7. Method according to one of claims 1 to 6, **characterized in that** in each case a value for the systolic blood pressure and/or a blood pressure amplitude and the diastolic blood pressure and/or a blood pressure amplitude is determined and output.

8. Device (1) for determining at least one circulation parameter of a test person, the device having a heartbeat measuring arrangement (2), a pulse measuring arrangement (3) and a circulation parameter determination unit (4), **characterized in that** the device (1) is embodied for carrying out a method according to one of claims 1 to 7.

## Revendications

1. Méthode de détermination d'au moins une valeur de tension artérielle d'une personne testée,

- dans laquelle un ECG d'un sujet est déterminé au moyen d'un dispositif ECG (2) et transmis à une unité de détermination des paramètres de circulation (4),
- dans lequel l'unité de détermination des paramètres de circulation (4) détermine au moins une valeur d'un tonus vagal à partir d'une évolution temporelle déterminée de l'ECG,
- dans lequel une valeur temporelle d'une période de pré-éjection est déterminée par l'unité de détermination des paramètres de circulation (4) au moyen d'au moins une valeur du tonus vagal,
- dans lequel un signal de pouls du sujet est déterminé au moyen d'un dispositif de mesure du pouls sur un vaisseau sanguin périphérique et transmis à l'unité de détermination des paramètres de circulation (4),

- dans lequel un temps d'arrivée du pouls est déterminé à partir de l'ECG et du signal de pouls au niveau du vaisseau sanguin périphérique, le temps d'arrivée du pouls étant le temps qui s'écoule entre un premier battement de coeur et l'apparition d'un premier signal de pouls associé au niveau du vaisseau sanguin périphérique,

- dans lequel un temps de transit corrigé de l'impulsion est déterminé en soustrayant la période de pré-éjection du temps d'arrivée de l'impulsion,

- dans lequel une valeur de longueur artérielle au niveau du vaisseau sanguin périphérique du sujet est déterminée et transmise à l'unité de détermination des paramètres de circulation (4),

- dans lequel une vitesse d'onde de pouls est déterminée par l'unité de détermination des paramètres de circulation (4) à partir de la longueur artérielle et du temps de transit corrigé de l'impulsion,

- dans lequel l'unité de détermination des paramètres de circulation (4) détermine au moins une valeur de pression sanguine à partir d'au moins une équation, laquelle équation comprend au moins la vitesse de l'onde de pouls en tant que variable et une pluralité de coefficients,

- dans lequel, lors d'une étape d'étalonnage, une pression sanguine de référence est mesurée directement sur le sujet et transmise à l'unité de détermination des paramètres de circulation (4), et dans lequel l'unité de détermination des paramètres de circulation (4) détermine les valeurs de la pluralité de coefficients en comparant la pression sanguine de référence mesurée et la vitesse de l'onde de pouls, ou

- dans lequel un groupe de valeurs relatives à au moins une caractéristique corporelle prédéterminable et/ou une habitude de vie du sujet est transmis à l'unité de détermination des paramètres de circulation (4), et dans lequel l'unité de détermination des paramètres de circulation (4) détermine la pluralité de coefficients en comparant les valeurs d'entrée avec des ensembles de données de comparaison stockés, les coefficients étant sélectionnés sur la base d'un degré prédéterminable de concordance et/ou de similitude des valeurs d'entrée avec les ensembles de données de comparaison stockés,

- la valeur de pression artérielle déterminée au moins une fois étant émise.

2. Méthode selon la revendication 1, **caractérisée par le fait que** la valeur du tonus végétatif est normalisée sur la base d'ensembles de données comparatives stockées avant d'être prise en compte dans la détermination de la valeur de la pression artérielle.

3. Méthode selon la revendication 1 ou 2, **caractérisée par le fait que** la valeur de la longueur artérielle du vaisseau sanguin périphérique du sujet est déterminée en mesurant la longueur correspondante.

4. Méthode selon l'une des revendications 1 à 3, **caractérisée par le fait que** l'au moins une caractéristique corporelle du sujet est au moins une caractéristique corporelle choisie dans le groupe : âge du sujet, sexe biologique du sujet, longueur du bras du sujet, taille du sujet, poids du sujet, origine ethnique, origine régionale.

5. Méthode selon l'une des revendications 1 à 4, **caractérisée par le fait que** l'au moins une habitude de vie du sujet est une habitude de vie choisie dans le groupe : consommation de drogues, usage de drogues, lieu de résidence, habitudes alimentaires, habitudes de sommeil/éveil.

6. Méthode selon l'une des revendications 1 à 5, **caractérisée par le fait qu'**une heure du jour ou de la journée ou une période de l'année est prise en compte lors de la détermination de la valeur de la pression artérielle, en particulier en ce que l'heure du jour ou de la journée ou la période de l'année à laquelle les ensembles de données de comparaison ont été créés est stockée pour chacun des ensembles de données de comparaison.

7. Méthode selon l'une des revendications 1 à 6, **caractérisée par le fait que**, dans chaque cas, une valeur pour la pression artérielle systolique et/ou une amplitude de pression artérielle et la pression artérielle diastolique et/ou une amplitude de pression artérielle est déterminée et émise.

8. Dispositif (1) pour déterminer au moins un paramètre de circulation d'une personne testée, le dispositif ayant un dispositif de mesure du rythme cardiaque (2), un dispositif de mesure du pouls (3) et une unité de détermination du paramètre de circulation (4), **caractérisé en ce que** le dispositif (1) est conçu pour mettre en oeuvre un procédé selon l'une des revendications 1 à 7.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 103598876 B
- US 20100125212 A1
- US 20120179382 A1
- AT 517071 B1
- WO 2015176088 A

**In der Beschreibung aufgeführte Nicht-Patentliteratur**

- **SUJAY DEB.** Cuff-Less Estimation of Blood Pressure using Pulse Transit Time and Pre-ejection Period. *Methode zur Blutdruckbestimmung unter Einbeziehung der PTT und der PEP*
- **J. MUEHLSTEFF.** *Cuffless Estimation of Systolic Blood Pressure for Short Effort Bicycle Tests: The Prominent Role oft he Pre-Ejection Period*
- **HYUN JAE BEAK et al.** *Enhancing the stimation of blood pressure using pulse arrival time and two confounding factors*